(19) Europäisches Patentamt
European Patent Office
Office européen des brevets

(11) **EP 3 864 399 B1**

(12) **FASCICULE DE BREVET EUROPEEN**

(45) Date de publication et mention
de la délivrance du brevet:
**06.09.2023   Bulletin 2023/36**

(21) Numéro de dépôt: **19829263.3**

(22) Date de dépôt: **04.11.2019**

(51) Classification Internationale des Brevets (IPC):
**G01N 21/85** (2006.01)        **G01N 21/71** (2006.01)

(52) Classification Coopérative des Brevets (CPC):
**G01N 21/718; G01N 21/8507**

(86) Numéro de dépôt international:
**PCT/FR2019/052594**

(87) Numéro de publication internationale:
**WO 2020/099758 (22.05.2020 Gazette 2020/21)**

(54) **DISPOSITIF DE CARACTÉRISATION D'UN MATÉRIAU LIQUIDE**

VORRICHTUNG ZUR CHARAKTERISIERUNG EINES FLÜSSIGEN MATERIALS

APPARATUS FOR CHARACTERISING A LIQUID MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.11.2018   FR 1860509**

(43) Date de publication de la demande:
**18.08.2021   Bulletin 2021/33**

(73) Titulaire: **Commissariat à l'énergie atomique et aux énergies alternatives**
**75015 Paris (FR)**

(72) Inventeurs:
• **BELRHITI, Younès**
**38054 GRENOBLE Cedex 09 (FR)**
• **ALBARIC, Mickaël**
**38054 GRENOBLE Cedex 09 (FR)**
• **BENMANSOUR, Malek**
**38054 GRENOBLE Cedex 09 (FR)**
• **PELLETIER, David**
**38054 GRENOBLE Cedex 09 (FR)**

(74) Mandataire: **Brevalex**
**Tour Trinity**
**1 B Place de la Défense**
**92400 Courbevoie (FR)**

(56) Documents cités:
**EP-A1- 3 023 771        EP-A2- 1 914 534**
**WO-A1-2015/177223       US-A- 4 802 656**
**US-A1- 2003 197 125**

• **RAI AWADHESH K ET AL: "High temperature fiber optic laser-induced breakdown spectroscopy sensor for analysis of molten alloy constituents", REVIEW OF SCIENTIFIC INSTRUMENTS, AIP, MELVILLE, NY, US, vol. 73, no. 10, 1 octobre 2002 (2002-10-01), pages 3589-3599, XP012039425, ISSN: 0034-6748, DOI: 10.1063/1.1505101 cité dans la demande**

## Description

## DOMAINE TECHNIQUE

La présente invention se rapporte au domaine général de la caractérisation d'un matériau liquide.

Elle concerne toute technique de caractérisation d'un matériau liquide et d'analyse de surface nécessitant une surface d'analyse représentative, stable et renouvelable du liquide. A titre d'exemples, l'invention peut être utilisée avec des techniques de spectrométrie comme la technique de spectrométrie de masse à plasma à couplage inductif (ICP-MS) et à d'autres techniques d'analyse de surface comme la pyrométrie. De façon préférentielle, l'invention est appliquée au domaine de l'analyse spectrale d'un matériau liquide mettant en oeuvre une technique de spectroscopie, et notamment la spectrométrie plasma induite par laser (encore appelée LIBS pour « Laser Induced Breakdown Spectroscopy » en anglais).

L'invention peut être appliquée à haute température ou à température ambiante. Par exemple, elle peut être appliquée à des matériaux à haute température comme les métaux fondus (aluminium, acier, zinc, sodium, ...), les sels fondus, les verres liquides dans un four de vitrification, et aux liquides à basse température comme l'eau, les huiles, entre autres.

L'invention peut être utilisée pour différentes applications telles que la purification des métaux, de l'eau, des huiles, de produits consommables liquides, entre autres.

L'invention est tout particulièrement concernée, mais non exclusivement, par le domaine de l'analyse spectrale en ligne par technique LIBS des métaux en fusion, et spécifiquement des métaux en fusion fortement oxydables, comme par exemple le silicium, l'aluminium ou le zirconium. Elle peut ainsi s'appliquer par exemple à l'analyse de la composition chimique du silicium liquide en fusion durant sa purification par des procédés métallurgiques, pour son utilisation ultérieure, par exemple dans l'industrie photovoltaïque.

L'invention propose ainsi un dispositif de caractérisation d'au moins un matériau liquide, un ensemble comportant une enceinte d'au moins un matériau liquide et un tel dispositif de caractérisation, ainsi qu'un procédé de caractérisation associé.

## ÉTAT DE LA TECHNIQUE ANTÉRIEURE

Dans le cadre de l'élaboration des cellules photovoltaïques, le silicium est le matériau le plus couramment utilisé. Il intervient ainsi dans la fabrication des cellules photovoltaïques dites « cristallines », c'est-à-dire qui sont à base de monocristaux de silicium ou de polycristaux de silicium.

Toutefois, la présence d'un taux élevé d'impuretés dans le silicium métallique liquide est néfaste pour l'effet photoélectrique parce qu'elle favorise la recombinaison des porteurs de charges, ce qui influence leur durée de vie et ainsi le rendement des cellules photovoltaïques. Aussi, il est primordial de pouvoir contrôler le taux d'impuretés du silicium.

Pour obtenir le matériau silicium à la pureté requise pour son application, il est possible d'utiliser des procédés métallurgiques (tels que par exemple la solidification dirigée, l'évaporation réactive, entre autres) dans lesquels le silicium métallurgique passe par une phase liquide en fusion, et est purifié par exploitation de propriétés physiques des impuretés du silicium (coefficients de partage entre phase liquide et phase solide ou liquide, propriétés de volatilité, par exemple) ou exploitation de propriétés de réactivité des impuretés du silicium (traitement par plasma, par exemple). Toutefois, afin d'obtenir le silicium à la pureté requise, la connaissance de la composition chimique du métal en fusion et la cinétique de purification doivent être maîtrisées et passent donc par une connaissance précise de l'évolution des concentrations en impuretés dans le matériau au cours du temps de traitement.

De façon habituelle, l'analyse des composants du matériau silicium au cours de son traitement de purification est réalisée par prélèvement, dans le métal en fusion, d'un échantillon à l'aide d'un contenant généralement en graphite dans lequel le silicium est solidifié, démoulé, puis envoyé pour son analyse. Néanmoins, cette procédure classique d'analyse du matériau n'est pas entièrement satisfaisante, et en particulier n'est pas adaptée pour le contrôle en continu de la pureté du métal en fusion, car elle exige des étapes de préparation préalables d'échantillons qui nécessitent du temps et présentent des coûts élevés.

Aussi, au vu de ces limitations, il est apparu souhaitable de pouvoir mettre au point un outil d'analyse en ligne *in situ* des composants du matériau silicium, et des métaux liquides, de façon à réduire ainsi le temps d'analyse, les coûts et à assurer un contrôle continu de la composition chimique du matériau au cours du temps. De la sorte, il est possible de n'avoir besoin d'aucun prélèvement du bain de métal liquide et de donner une information en temps réel sur sa composition.

Pour ce faire, dans la perspective de l'analyse chimique et du diagnostic d'un matériau donné, le laser constitue un outil privilégié car il permet d'effectuer des opérations de détection et d'identification dans des conditions d'environnement très variées. De plus, les mesures réalisées par une technique laser présentent de nombreux atouts, et peuvent permettre une analyse *in situ,* sans prélèvement ni contact, ainsi qu'une rapidité d'acquisition des informations et l'utilisation pour des analyses locales ou à distance.

Parmi les techniques existantes utilisant un laser, la spectrométrie plasma induite par laser (dénommée « technique LIBS ») constitue une méthode analytique physique bien connue, utilisée pour l'analyse des constituants d'un matériau afin de le caractériser, et représente une technologie parmi les plus prometteuses pour le contrôle en ligne de la composition d'un bain métallique en fusion. La technique LIBS est typiquement utilisée pour permettre une analyse rapide, directe (sans préparation d'échantillons) et en ligne de matériaux sous forme solide, liquide ou gazeuse. Elle met ainsi en oeuvre l'ablation-laser d'un matériau pour créer un plasma, puis la technique spectroscopique pour l'observation et l'analyse du spectre d'émission lumineuse du plasma afin de déterminer les composants du matériau.

Plus précisément, la technique LIBS implique la focalisation d'une impulsion laser vers la surface du matériau à analyser, qui provoque la formation d'un micro-plasma. Ce micro-plasma se forme quasiment de façon immédiate, c'est-à-dire alors que l'impulsion laser n'est pas terminée. A la fin de l'impulsion laser, les espèces atomiques et ioniques du micro-plasma se désexcitent et réémettent alors un rayonnement qu'un analyseur, i.e. un spectromètre, capte et traduit afin d'obtenir un spectre décrivant les espèces chimiques qui composent le matériau.

Ainsi, la technique LIBS peut permettre l'identification, grâce à la longueur d'onde d'émission, et la quantification, grâce à l'intensité d'émission, des composants présents dans le matériau à analyser. De plus, la technique LIBS permettant de réaliser une analyse à distance, elle s'avère tout particulièrement adaptée pour l'analyse des matériaux à l'état fondu à haute température, et notamment pour l'analyse du silicium en fusion, et l'analyse des matériaux ne pouvant pas être manipulés car représentant un danger potentiel. Aussi, dans le cas des métaux en fusion, tels que le silicium présenté précédemment, la technique LIBS est susceptible de fournir en temps réel l'évolution de la composition chimique du matériau, et notamment la teneur en impuretés du silicium métallurgique en fusion au cours de ses différentes étapes de purification.

Diverses solutions ont ainsi déjà été envisagées dans l'art antérieur pour mettre au point l'analyse en ligne de métaux en fusion par le biais de la technique LIBS.

Ainsi, dans l'article intitulé « Impurity détection in solid and molten silicon by laser induced breakdown spectroscopy », Sarah Darwiche et al, 2012, Spectrochimica Acta Part B, Volume 74-75, pages 115-118, les auteurs ont énoncé le principe de base consistant à focaliser une impulsion laser sur la surface d'un bain de métal en fusion, en particulier du silicium, et à collecter le signal émis par le plasma généré à l'aide d'un détecteur. Le dispositif optique de mesure et le laser sont alors placés à distance suffisante du bain de métal en fusion afin d'éviter un endommagement éventuel par les flux de chaleur. Dans le cadre de cet article, l'analyse du silicium en fusion a été réalisée à distance de manière non intrusive et sur de faibles quantités de matière, en milieu inerté avec de l'argon. Néanmoins, à plus grande échelle, cette méthode de mesure peut présenter un inconvénient majeur lié au fait que, dans le cas de métaux en fusion, la composition chimique de la surface analysée n'est généralement pas représentative de la composition du volume global du métal. En effet, de par la forte réactivité des métaux en fusion, et par exemple du silicium, le matériau présent à l'interface avec l'atmosphère subit généralement des phénomènes d'oxydation ou de nitruration qui conduisent à la génération de laitiers de surface. Du fait de la ségrégation des diverses impuretés entre le métal fondu et la phase oxydée, le laitier n'a généralement pas la même composition que le métal, ce qui remet donc en cause la fiabilité des mesures réalisées avec cette méthode.

Des systèmes d'analyse alternatifs ont également été développés afin de créer une surface d'analyse sur l'échantillon qui soit représentative du volume de métal en fusion. Dans l'article intitulé « High température fiber optic laser-induced breakdown spectroscopy sensor for analysis of molten alloy constituents », Awadhesh K. Rai et al, octobre 2002, Review of Scientific Instruments, Volume 73, pages 3589-3599, n°10, il est présenté un dispositif d'analyse par technique LIBS qui permet d'approcher la tête de mesure à proximité d'un bain d'aluminium en fusion et de créer une surface libre pour l'analyse à l'aide d'une entretoise. Le dispositif de collecte est protégé par une coque en acier inoxydable. Néanmoins, ce dispositif n'est pas satisfaisant en ce qu'il présente notamment l'inconvénient de limiter l'analyse à des matériaux à bas point de fusion, typiquement de l'ordre de 660°C pour l'aluminium à la pression atmosphérique, alors que le silicium, par exemple, présente un point de fusion aux alentours de 1412°C à la pression atmosphérique.

La demande internationale WO 02/063284 A2 propose encore une autre solution qui prévoit de faire circuler le métal liquide à travers une cellule et d'en faire l'analyse de surface par technique LIBS au moyen d'un accès optique. Cette solution a été appliquée pour le zinc et l'aluminium, mais elle n'est pas efficace pour des métaux très oxydables comme le silicium ou le zirconium. En effet, dans le cas d'un métal fortement oxydable, une oxydation de surface peut se produire avec l'oxygène résiduel de la charge et rendre l'analyse de surface invalide.

La demande de brevet US 2003/0234928 A1 décrit quant à elle une solution complémentaire à la précédente, pour les métaux très oxydables. Selon son principe, l'extrémité d'un tube est plongée sous la surface du métal liquide à travers duquel un bullage par gaz inerte est réalisé pour permettre de renouveler la surface du métal liquide et de générer un volume d'analyse dans une atmosphère neutre. L'information optique du plasma est alors collectée par l'intermédiaire d'un jeu de miroirs et de fibres optiques. Toutefois, cette solution présente l'inconvénient majeur d'une instabilité de la surface à analyser qui est intimement liée à la dynamique de la bulle générée. Une synchronisation, délicate à réaliser, entre la fréquence d'analyse et la dynamique de formation de la bulle est alors nécessaire. En outre, la note technique intitulée « VAI-CON® Chem-A New Continuous Chemical Analysis System of Liquid Steel in Metallurgical Vessels », N. Ramaseder et al, février 2004, La metallurgia italiana, pages 60-63, présente une solution similaire dans le cadre de l'analyse d'un bain d'acier. Cependant, cette solution a été testée sur des fours pilotes et reste difficilement applicable à des fours de capacité industrielle car elle utilise un jeu de miroirs dont le réglage est très pointu.

La demande de brevet français FR 3 021 407 A1 décrit par ailleurs un dispositif d'analyse d'un métal en fusion oxydable par technique LIBS pourvu d'un système de brassage permettant de renouveler localement la surface du métal fondu et qui est relié à un guide d'ondes permettant de focaliser le faisceau laser à la surface du liquide. Ainsi, le dispositif permet d'obtenir des mesures rapides, de meilleures limites de détection et une analyse plus robuste.

Il existe toutefois un besoin pour améliorer encore les dispositifs connus d'analyse de métaux en fusion par technique LIBS, et notamment pour disposer d'un brassage efficace permettant l'obtention d'une surface d'analyse renouvelable, stable et représentative du bain métallique.

Dans l'industrie métallurgique, différentes formes de pales de brassage mécanique existent et ont été conçues pour différentes applications. En effet, certaines pales sont utilisées pour brasser le bain et faciliter sa purification en dissolvant des éléments métalliques et en dispersant des particules gazeuses dedans.

A titres d'exemples, le brevet américain US 4,717,540 A divulgue un système de brassage qui permet de faciliter la dissolution des particules de nickel, dont la température de fusion est élevée, dans un bain de zinc et d'éviter la formation de masses de nickel semi-plastiques ou l'agglomération du zinc avec ces masses au fond du bain. Ce système de brassage est composé d'un arbre rotatif creux qui permet l'introduction d'un gaz inerte au mélange par le biais d'orifices. Le système contient une grille d'orifices qui permet de bloquer le passage des particules de nickel, introduites par un tube, non dissoutes. La vitesse de rotation retenue est celle qui permet de générer un vortex qui va empêcher la réalisation de tout type d'analyse ou contrôle *in situ* en surface nécessitant une stabilité de mesure.

En outre, le brevet américain US 8,281,964 B2 décrit l'introduction d'un gaz inerte, éventuellement avec des particules métalliques, dans le métal liquide pour éliminer l'hydrogène ainsi que les inclusions d'oxyde et en même temps obtenir un brassage rapide et efficace. Le gaz atteint le métal liquide en passant par un tube d'entraînement creux qui passe par un passage rotatif couplé à un moteur excentré et qui est relié à l'arbre rotatif creux par une bride et un coupleur. Le système comprend une plaque permettant de réguler l'écoulement du bain d'aluminium pendant le brassage, cette plaque jouant le rôle d'une chicane. Les paramètres de rotation sont choisis de manière à générer temporairement un vortex. La géométrie des pales a été conçue de telle manière à bien mélanger rapidement le gaz avec le métal dans la chambre à l'intérieur de la pale qui contient des ouvertures, d'entrée et de sortie, et ainsi permettre un dégazage efficace sachant que la vitesse de rotation peut être réduite tout en gardant la même efficacité. Cette technique permet d'avoir un brassage et un dégazage efficace. Néanmoins, la présence du vortex va empêcher la réalisation des mesures de contrôle *in situ* dans le bain suite à l'absence d'une surface d'analyse stable.

Par ailleurs, le brevet américain US 4,802,656 A enseigne encore la conception de pales permettant de faciliter la dissolution des additifs métalliques (Fe, Mg, Si, Mn, ...) dans le bain d'aluminium qui permettent d'améliorer les propriétés de l'aluminium, et notamment les caractéristiques mécaniques d'un côté. D'un autre, ces pales ont permis de mieux disperser le gaz chloré dans le bain, par le biais d'orifices internes, pour éliminer les impuretés alcalines et alcalino-terreux qui sont derrière l'augmentation du taux d'oxydation du métal liquide ou forment des composés qui peuvent dégrader leurs propriétés à haute température. La problématique résidait dans la rétention du gaz qui a tendance à s'échapper rapidement vers le haut pour rejoindre l'atmosphère et le maintien des métaux qui eux, au contraire, tombent facilement au fond du bain et s'agglomèrent. Contrairement aux nombreuses pales utilisées pour des applications à hautes températures, la géométrie de la pale a ici été conçue pour permettre l'obtention d'un brassage en absence de zones de turbulence et de vortex, pour avoir un brassage global de tout le liquide du bain, pour la génération d'un brassage efficace avec une faible vitesse de rotation, pour une dissémination des bulles de gaz à travers les orifices internes pour empêcher leur remontée. Néanmoins, la géométrie de la pale sert à obtenir un brassage efficace à faible vitesse de rotation et ne garantit pas la présence d'une zone d'analyse stable, renouvelable et représentative qui pourrait être destinée à réaliser des mesures de caractérisation *in situ*.

Aussi, les différentes pales de l'art antérieur permettent de réaliser un brassage, de dissoudre des éléments métalliques en même temps et de disperser des particules gazeuses. Toutefois, elles ne sont pas destinées à être utilisées pour garantir le renouvellement, la reproductibilité et la stabilité d'une surface qui peut être analysée par une technique de caractérisation *in situ* telle que par exemple la technique LIBS.

## EXPOSÉ DE L'INVENTION

Il existe par conséquent un besoin pour proposer un dispositif de caractérisation en ligne, notamment par technique LIBS, de matériaux liquides, notamment des métaux en fusion oxydables, et particulièrement fortement oxydables, comprenant un système de brassage permettant l'obtention d'une surface stationnaire d'analyse, renouvelable et représentative du bain ou du volume, permettant de simplifier l'analyse, de réduire le temps d'analyse, de réduire les coûts et d'assurer un suivi qualité des matériaux. Par « surface stationnaire », il faut comprendre que les propriétés de la surface servant à l'analyse sont reproductibles dans le temps (à savoir notamment son niveau, sa forme, sa composition, sa vitesse d'écoulement, entre autres). En particulier, la préparation et le renouvellement de la surface servant à l'analyse doivent pouvoir assurer l'évacuation d'une tierce phase (laitier, oxyde, nitrure, par exemple) qui pourrait invalider les résultats de l'analyse du matériau.

L'invention a donc pour but de répondre au moins partiellement aux besoins mentionnés ci-dessus et de remédier aux inconvénients relatifs aux réalisations de l'art antérieur.

L'invention a ainsi pour objet, selon l'un de ses aspects, un dispositif de caractérisation, notamment d'analyse, d'au moins un matériau liquide, notamment un métal en fusion, en particulier oxydable, préférentiellement par technique de spectroscopie, notamment par technique LIBS, comportant :

- des moyens de caractérisation, notamment d'analyse par technique de spectroscopie, notamment par technique LIBS,
- des moyens de brassage mécanique rotatifs d'un bain liquide dudit au moins un matériau liquide, les moyens de brassage mécanique comportant :

  - une partie centrale, s'étendant selon un axe longitudinal, destinée à être positionnée au-dessus du bain liquide dudit au moins un matériau liquide, comprenant une cavité interne formant une chambre d'analyse délimitée par une paroi de la partie centrale, la partie centrale comportant une première extrémité reliée aux moyens de caractérisation, et
  - une pluralité de pales de brassage mécanique, ou encore une pluralité de moyens de mélange et/ou de brassage, reliées à une deuxième extrémité de la partie centrale, opposée à la première extrémité de la partie centrale, la deuxième extrémité de la partie centrale s'étendant sur une hauteur selon l'axe longitudinal de la partie centrale et étant creuse de sorte à assurer une communication fluidique entre la cavité interne et le bain liquide dudit au moins un matériau liquide,

  les moyens de caractérisation étant configurés pour permettre l'analyse de la surface dudit au moins un matériau liquide, situé dans la portion au droit de la cavité interne de la partie centrale, caractérisé en ce que les pales de brassage mécanique sont destinées à être totalement immergées dans le bain liquide dudit au moins un matériau liquide,
  en ce que la partie centrale comporte un ou plusieurs orifices formés de façon traversante dans sa paroi délimitant la cavité interne et situés au-dessus de la deuxième extrémité de la partie centrale portant les pales de brassage mécanique lorsque le dispositif est en place dans le bain liquide dudit au moins un matériau liquide, le ou les orifices étant destinés à être partiellement immergés selon une hauteur dans le bain liquide dudit au moins un matériau liquide, et en ce que chaque pale de brassage mécanique comporte au moins une aile de brassage orientée selon un angle d'orientation non nul par rapport à un axe transversal perpendiculaire à l'axe longitudinal de la partie centrale.

De façon préférentielle, le dispositif selon l'invention permet la caractérisation d'au moins un matériau liquide, et notamment l'analyse d'au moins un métal en fusion, notamment oxydable, et tout particulièrement par technique LIBS. Cependant, l'invention s'applique également à toute technique de caractérisation d'au moins un matériau liquide nécessitant une analyse *in situ* et un besoin de réaliser des mesures sur une surface d'analyse stable, représentative et renouvelable du matériau liquide. Ainsi, par exemple, l'invention peut utiliser une technique de spectrométrie de masse, comme la technique de spectrométrie de masse à plasma à couplage inductif (ou technique ICP-MS pour « Inductively Coupled Plasma Mass Spectrometry » en anglais). Cette technique ICP-MS est capable de détecter les métaux et plusieurs non métaux à des concentrations très faibles. L'invention, couplée à différentes techniques de caractérisation telles que la technique ICP-MS, peut ainsi être utilisée dans plusieurs applications industrielles telles que l'analyse de la contamination de l'eau.

Grâce à l'invention, il peut ainsi être possible d'améliorer encore l'analyse en ligne de matériaux, et notamment l'analyse en ligne par technique LIBS de métaux en fusion, tout spécialement adaptée aux métaux oxydables, et notamment fortement oxydables, en comparaison avec les solutions de l'art antérieur présentées précédemment. En effet, le dispositif selon l'invention permet avantageusement un renouvellement de la surface à analyser par mélange mécanique à l'aide des pales de brassage, la stabilité de la surface d'analyse et la représentativité de la surface d'analyse. Il prévoit également la présence d'une chambre d'analyse spécifique pour permettre notamment la focalisation de l'impulsion laser utilisée par exemple dans la technique LIBS.

Le dispositif selon l'invention peut en outre comporter l'une ou plusieurs des caractéristiques suivantes prises isolément ou suivant toutes combinaisons techniques possibles.

Selon une première variante, les pales de brassage mécanique peuvent s'étendre, depuis la paroi externe de la deuxième extrémité de la partie centrale, en éloignement de la cavité interne de la partie centrale.

Selon une deuxième variante, les pales de brassage mécanique peuvent s'étendre, depuis la paroi interne de la deuxième extrémité de la partie centrale, à l'intérieur de la cavité interne de la partie centrale.

Par ailleurs, la hauteur de chaque pale de brassage mécanique, selon l'axe longitudinal de la partie centrale, peut être sensiblement égale à la hauteur, selon l'axe longitudinal de la partie centrale, de la deuxième extrémité de la partie centrale.

De plus, l'angle d'orientation peut être inférieur ou égal à 20°.

Les moyens de brassage mécanique peuvent préférentiellement comporter trois pales de brassage mécanique et la partie centrale peut comporter trois orifices séparés les uns des autres par des portions de paroi longitudinales.

Le ou les orifices peuvent être destinés à être partiellement immergés selon une hauteur dans le bain liquide dudit au moins un matériau liquide, au moins égale à un quart de la hauteur totale du ou des orifices.

Avantageusement, la présence de tels orifices sur une portion de la partie centrale située au-dessus des pales de brassage mécanique peut permettre d'assurer le renouvellement de la surface à analyser. En effet, le matériau liquide présent dans la portion au droit de la cavité interne de la partie centrale peut voir son niveau remonter le long de la paroi de la partie centrale délimitant la cavité interne, en particulier sous l'effet de la centrifugation et de la capillarité. De cette façon, la présence d'orifices permettant l'écoulement permanent en dehors de la cavité interne de l'excès de matériau liquide qui remonte le long de la paroi, peut permettre de garantir le renouvellement et la stabilisation du niveau de la surface à analyser.

Par ailleurs, la partie centrale peut se présenter sous la forme d'un tube (ou arbre) creux, notamment sous la forme d'un tube cylindrique creux.

En particulier, la deuxième extrémité de la partie centrale, sur laquelle sont fixées les pales de brassage mécanique, est avantageusement creuse de sorte à assurer une circulation verticale du matériau liquide.

Les moyens de caractérisation peuvent être compris dans une tête d'analyse située au niveau de la première extrémité de la partie centrale. De plus, les moyens de brassage mécanique peuvent former ensemble un brasseur mécanique couplé à la tête d'analyse.

La forme, les dimensions et/ou la vitesse de rotation des pales de brassage mécanique et/ou de la partie centrale peuvent être déterminées et ajustées en fonction de la stabilisation requise de la surface à analyser.

Les dimensions des pales de brassage mécanique peuvent être définies en fonction des dimensions de l'enceinte dans laquelle est située ledit au moins un matériau liquide.

De façon avantageuse, la partie centrale peut jouer le rôle de chambre d'analyse et permettre la focalisation d'une impulsion laser envoyée par les moyens de caractérisation sous forme de moyens d'analyse par technique de spectroscopie vers la surface à analyser, et permettre aussi le confinement du micro-plasma qui se forme.

De façon préférentielle, la partie centrale peut être réalisée en graphite. De plus, la partie centrale peut être revêtue extérieurement d'une couche formant barrière de passivation, notamment vis-à-vis du silicium, par exemple une couche de carbure de silicium (SiC).

En outre, l'invention a encore pour objet, selon un autre de ses aspects, un ensemble, caractérisé en ce qu'il comporte :

- une enceinte comportant un bain d'au moins un matériau liquide, notamment d'au moins un métal en fusion, en particulier oxydable,
- un dispositif de caractérisation tel que défini précédemment, pour la caractérisation dudit au moins un matériau liquide de l'enceinte.

Ledit au moins un matériau liquide peut avantageusement être un métal en fusion, notamment un métal fortement oxydable, étant notamment choisi parmi le silicium ou le zirconium.

Ledit au moins un matériau liquide peut encore être un matériau à haute température comme un métal fondu, par exemple aluminium, acier, zinc, sodium, entre autres, un sel fondu, un verre liquide dans un four de vitrification et/ou un matériau à basse température comme l'eau, une huile, entre autres.

L'enceinte peut être de forme cylindrique présentant un diamètre interne, les pales de brassage mécanique peuvent présenter une plus grande dimension transversale, notamment un diamètre, selon un axe transversal perpendiculaire à l'axe longitudinal de la partie centrale. La relation suivante peut être vérifiée :

$$0,2 < Dp/De < 0,7,$$

où :

Dp est la plus grande dimension transversale des pales de brassage mécanique, et
De est le diamètre interne de l'enceinte.

L'enceinte peut également être dénommée creuset. Elle peut par exemple être réalisée en graphite.

Par ailleurs, l'ensemble peut également comporter des moyens de chauffage de l'enceinte comportant ledit au moins un matériau liquide.

L'enceinte et la partie centrale du dispositif de caractérisation peuvent être préférentiellement réalisées dans un même matériau, notamment en graphite.

Le régime d'écoulement dudit au moins un matériau liquide situé dans la portion au droit de la cavité interne de la partie centrale est avantageusement laminaire, cet écoulement étant caractérisé par un nombre de Reynolds $Re$ compris entre 100 et 5000, et notamment entre 1000 et 2000, ce nombre de Reynolds $Re$ étant donné par la formule suivante :

$$Re = [(\omega \times R) \times R']/v,$$

où :

- ($\omega \times R$) représente la vitesse caractéristique de l'écoulement, à savoir le produit de la vitesse angulaire de rotation $\omega$ d'une pale de brassage mécanique et de la distance R entre l'extrémité de la pale de brassage mécanique et l'axe de la partie centrale,
- R' représente une dimension caractéristique de l'écoulement, à savoir notamment le rayon de la partie centrale dans le cas où elle est cylindrique ou le côté de la partie centrale dans le cas où elle est carrée par exemple, et
- v représente la viscosité cinématique du liquide.

Ainsi, le choix de la valeur du nombre de Reynolds *Re* effectué pour caractériser l'écoulement dudit au moins un matériau liquide situé dans la portion au droit de la cavité interne de la partie centrale peut donc avoir un impact direct sur les paramètres caractérisant l'intensité du brassage mécanique du bain de matériau liquide.

Par ailleurs, l'invention a aussi pour objet, selon un autre de ses aspects, un procédé de caractérisation, notamment d'analyse, d'au moins un matériau liquide, notamment d'au moins un métal en fusion, en particulier oxydable, préférentiellement par technique de spectroscopie, notamment par technique LIBS, caractérisé en ce qu'il est mis en oeuvre au moyen d'un dispositif de caractérisation tel que défini précédemment, et en ce qu'il comporte les étapes simultanées consistant à :

- effectuer un brassage mécanique d'un bain liquide dudit au moins un matériau liquide par le biais des moyens de brassage mécanique rotatifs du dispositif,
- analyser, notamment par technique de spectroscopie, la surface dudit au moins un matériau liquide situé dans la portion au droit de la cavité interne de la partie centrale par le biais des moyens de caractérisation.

Ledit au moins un matériau liquide peut être un métal en fusion oxydable, notamment un métal fortement oxydable, étant notamment choisi parmi le silicium ou le zirconium, les moyens de caractérisation peuvent être des moyens d'analyse par technique de spectroscopie comprenant des moyens d'analyse par technique LIBS, et le procédé peut comporter au moins une étape d'analyse en ligne par technique LIBS d'une ou plusieurs impuretés contenues dans ledit au moins un métal en fusion oxydable, notamment du silicium, lors d'un processus de purification dudit au moins un métal en fusion oxydable.

Le dispositif, l'ensemble et le procédé selon l'invention peuvent comporter l'une quelconque des caractéristiques énoncées dans la description, prises isolément ou selon toutes combinaisons techniquement possibles avec d'autres caractéristiques.

**BRÈVE DESCRIPTION DES DESSINS**

L'invention pourra être mieux comprise à la lecture de la description détaillée qui va suivre, d'exemples de mise en oeuvre non limitatifs de celle-ci, ainsi qu'à l'examen des figures, schématiques et partielles, du dessin annexé, sur lequel :

- la figure 1 représente, en coupe, un exemple de dispositif d'analyse d'un métal en fusion oxydable par technique LIBS conforme à l'invention, et
- les figures 2 et 3 représentent, partiellement et en perspective, deux exemples de réalisation de la partie centrale et des pales de brassage mécanique d'un dispositif d'analyse d'un métal en fusion oxydable par technique LIBS conforme à l'invention.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

**EXPOSÉ DÉTAILLÉ DE MODES DE RÉALISATION PARTICULIERS**

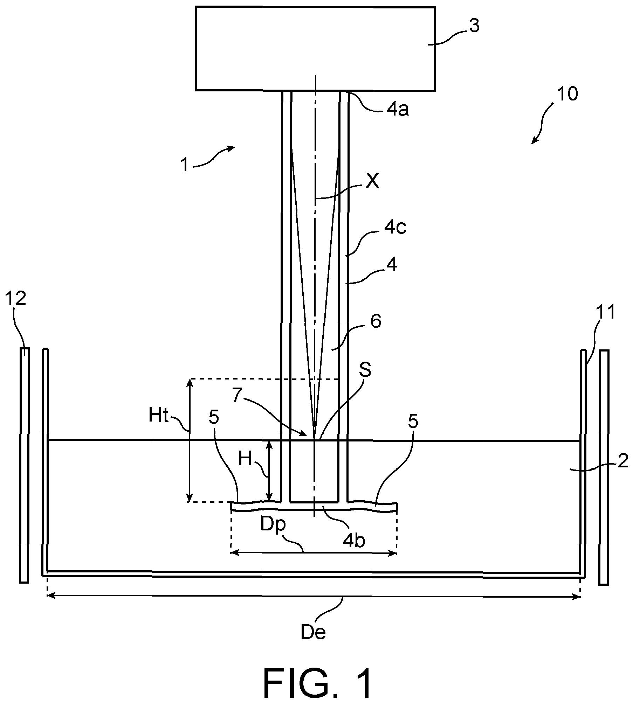

Dans l'exemple décrit ci-après en référence à la figure 1, on considère que l'invention s'applique à l'analyse d'un métal en fusion oxydable par technique LIBS. Toutefois, l'invention pourrait aussi s'appliquer à l'analyse d'un matériau liquide, par exemple de l'eau, par une autre technique de spectroscopie, par exemple une technique de spectrométrie de masse, comme la technique de spectrométrie de masse à plasma à couplage inductif (ou technique ICP-MS).

De plus, on considère ici que le métal 2 en fusion oxydable correspond à du silicium, et notamment du silicium métallurgique. Le dispositif 1 d'analyse selon l'invention peut alors être utilisé pour le contrôle en continu des concentrations des impuretés contenues dans ce silicium liquide en fusion, lors d'un processus de purification du métal visant par exemple à permettre une utilisation ultérieure pour l'élaboration de cellules photovoltaïques. Bien entendu, ce choix n'est nullement limitatif. En particulier, l'invention pourrait avantageusement s'appliquer à d'autres types de métaux en fusion oxydables, et notamment fortement oxydables, comme par exemple le zirconium, dont l'analyse de surface nécessite un renouvellement permanent de la matière afin de garantir une fiabilité acceptable des résultats de mesure.

On se réfère ainsi à la figure 1 qui représente, en coupe, un exemple de dispositif 1 d'analyse d'un métal 2 en fusion oxydable, à savoir le silicium, par technique LIBS conforme à l'invention.

Conformément à l'invention, et comme décrit dans la demande de brevet français FR 3 021407 A1, le dispositif 1 d'analyse comporte des moyens d'analyse par technique LIBS 3 et des moyens de brassage mécanique 4, 5 rotatifs d'un bain liquide de silicium 2 en fusion. Ce bain liquide de silicium 2 comporte par exemple une charge de silicium de grade métallurgique amélioré (encore dénommée UMG-Si pour « Upgraded metallurgical-Grade Silicon » en anglais), qui comporte une composition chimique d'environ 300 ppm en masse de métaux tous confondus, environ 15 ppm en masse de bore et environ 20 ppm en masse de phosphore.

Plus spécifiquement, le dispositif 1 d'analyse est utilisé dans un ensemble 10 conforme à l'invention qui comporte, en plus du dispositif 1 d'analyse, une enceinte 11, couramment dénommée creuset, et par exemple réalisée en silice et revêtue de nitrure de silicium, comprenant le bain de silicium 2 liquide en fusion. En particulier, l'utilisation du dispositif 1 d'analyse selon l'invention peut être faite dans cet exemple sur une opération de ségrégation dans un four de solidification dirigée d'une capacité d'environ 60 kg. En outre, afin de permettre le chauffage du silicium 2 dans l'enceinte 11 et le maintien d'une température supérieure à sa température de fusion d'environ 1412 °C à la pression atmosphérique, des moyens de chauffage résistifs 12 de l'enceinte 11 sont également prévus. De cette façon, la charge de silicium 2 peut par exemple être fondue sous un flux d'argon par chauffage résistif.

Par ailleurs, les moyens de brassage mécanique 4, 5 forment ensemble un brasseur mécanique 4, 5, couplé aux moyens d'analyse par technique LIBS 3 situés dans une tête d'analyse LIBS 3.

Ce brasseur mécanique 4, 5 comporte une partie centrale 4 partiellement immergée dans le bain liquide de silicium 2 en fusion, qui comprend une cavité interne 6 formant une chambre d'analyse. De plus, la partie centrale 4 comporte une première extrémité 4a qui est reliée à la tête d'analyse LIBS 3.

La partie centrale 4 se présente par exemple sous la forme d'un tube de brassage creux cylindrique, pourvu d'une paroi annulaire 4c délimitant la cavité interne 6, et présente par exemple un diamètre intérieur d'environ 25 mm et un diamètre extérieur d'environ 65 mm.

La partie centrale 4 joue le rôle de chambre d'analyse et permet la focalisation de l'impulsion laser envoyée par la tête d'analyse LIBS 3 vers la surface S à analyser du bain de silicium 2. Cette partie centrale 4 est notamment réalisée en graphite, et revêtue extérieurement d'une couche formant barrière de passivation vis-à-vis du silicium, par exemple une couche de carbure de silicium.

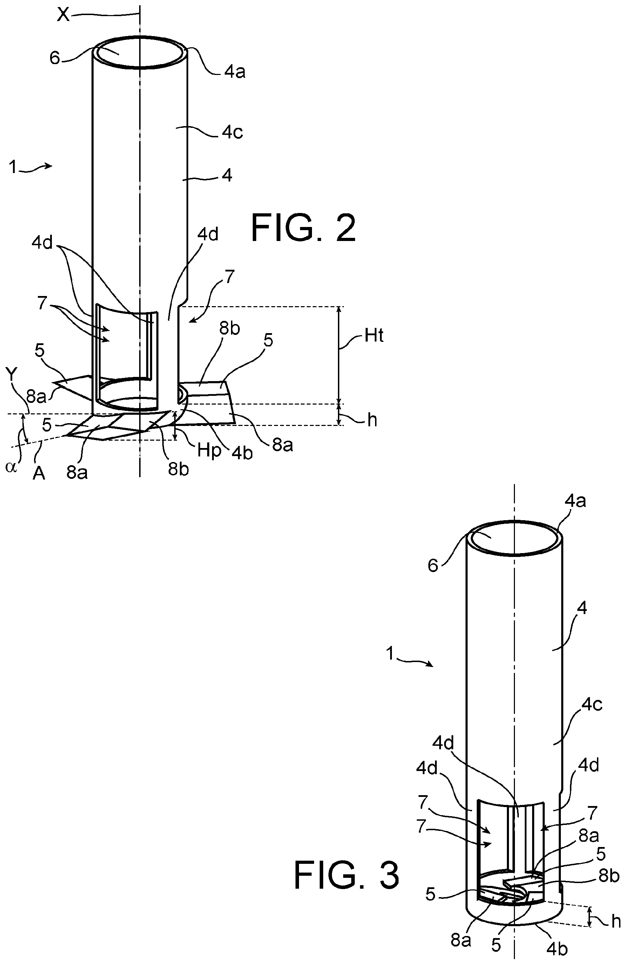

De plus, le brasseur mécanique 4, 5 comporte également des pales de brassage mécanique 5 immergées totalement dans le bain de silicium 2, et reliées à une deuxième extrémité 4b de la partie centrale 4, avantageusement creuse de sorte à assurer une communication fluidique entre la cavité interne 6 et le bain de silicium 2. Les pales de brassage mécanique 5 peuvent être réalisées de différentes manières, leur représentation étant très schématique sur la figure 1. Les figures 2 et 3, décrites par la suite, permettent d'envisager deux modes de réalisation distincts des pales 5.

Par ailleurs, la partie centrale 4 comporte des orifices 7 formés dans sa paroi annulaire 4c, mieux visibles sur les figures 2 et 3, qui sont situés au-dessus des pales de brassage mécanique 5 lorsque le dispositif 1 est en place dans le bain liquide de silicium 2. Ces orifices 7 permettent d'assurer le renouvellement de la surface S à analyser et le maintien du niveau de silicium 2 à un niveau constant dans la cavité interne 6. Une hauteur H de ces orifices 7 est totalement immergée dans le bain liquide de silicium 2, ce qui implique une immersion totale des pales de brassage mécanique 5.

Ainsi, les orifices 7 de la partie centrale 4 sont partiellement immergés dans le bain de silicium liquide 2, d'une hauteur H, et ils présentent une largeur suffisante pour assurer une circulation horizontale du silicium liquide 2 et pas uniquement une évacuation de l'excès de métal.

Lorsque le silicium 2 est en fusion complète, la partie centrale 4 est introduite progressivement dans le bain de silicium 2 avec immersion totale des pales de brassage 5, puis grâce à un moteur, elle est mise en rotation pour assurer le brassage du bain de silicium 2. L'analyse par technique LIBS est alors réalisée sur la surface S du silicium 2 situé dans la portion au droit de la partie centrale 4.

La rotation de la partie centrale 4 peut par exemple se faire à l'aide de l'empilement d'au moins deux pignons fixés autour de l'axe central X, ou axe longitudinal, de la partie centrale 4. La vitesse de rotation du brasseur mécanique 4, 5 peut être fixée de sorte à obtenir un écoulement laminaire du silicium 2 au droit de la partie centrale 4, et une circulation facilitée du silicium 2 au travers des orifices 7 prévus sur la partie centrale 4.

De façon plus générale, il est avantageux d'obtenir un régime d'écoulement du silicium 2 situé au droit de la cavité interne 6 de la partie centrale 4 qui soit laminaire. Un écoulement laminaire est souhaité dans la partie creuse de la pale jusqu'à la surface d'analyse qui doit être stable, sans turbulence. En dehors de cette zone, on souhaite un brassage efficace et il est possible que l'écoulement ne soit pas laminaire.

Pour ce faire, le nombre de Reynolds $Re$ est préférentiellement compris entre 100 et 5000, et notamment entre 1000 et 2000, ce nombre de Reynolds $Re$ étant donné par la formule suivante : $Re = [(\omega \times R) \times R']/v$, dans laquelle : $(\omega \times R)$ représente la vitesse caractéristique de l'écoulement, à savoir le produit de la vitesse angulaire de rotation $\omega$ d'une pale de brassage mécanique 5 et de la distance R entre l'extrémité de la pale de brassage mécanique 5 et l'axe X de la partie centrale 4, R' représente une dimension caractéristique de l'écoulement, à savoir le rayon de la partie centrale 4, et v représente la viscosité cinématique du liquide.

Le renouvellement de la surface d'analyse S, ciblée par le laser traversant la partie centrale 4 creuse reliée à la tête d'analyse 3 LIBS, est garanti par l'utilisation des orifices 7 au niveau de la partie centrale 4. Ces orifices 7 présentent une hauteur H qui est totalement immergée dans le bain liquide de silicium 2, ce qui entraîne une immersion totale des pales de brassage 5.

La deuxième extrémité 4b de la partie centrale 4, sur laquelle les pales de brassage 5 sont fixées, est avantageusement creuse pour pouvoir assurer une circulation verticale du métal liquide 2 vers le haut et plus précisément vers la surface à analyser S ciblée par le laser de la tête 3 LIBS. Le diamètre de cette deuxième extrémité 4b peut varier en fonction du diamètre de l'enceinte 11 contenant le silicium liquide 2. Par ailleurs, la hauteur h de la deuxième extrémité 4b, visible sur les figures 2 et 3, est de l'ordre de 6 mm.

La stabilité de la surface d'analyse S est obtenue par un brassage à des vitesses de rotation modérées qui évitent la formation de vortex ou des zones de turbulence ou des cavités.

La surface d'analyse S est représentative étant donné que le brassage est efficace et permet une circulation du silicium liquide 2 dans l'ensemble du bain. L'efficacité du brassage est obtenue par le biais de paramètres de brassage, tels que vitesse et sens de rotation, positionnement des pales 5, entre autres, et par le biais de la forme des pales de brassage 5. Ces pales peuvent être disposées à l'intérieur de la deuxième extrémité 4b, comme décrit par la suite en référence à la figure 3, ou à l'extérieur, comme décrit par la suite en référence à la figure 2.

Les figures 2 et 3 représentent, partiellement et en perspective, deux exemples de réalisation de la partie centrale 4 (la tête d'analyse 3 LIBS n'étant pas représentée) et des pales de brassage mécanique 5 d'un dispositif d'analyse 1 de silicium 2 en fusion par technique LIBS.

L'exemple de réalisation de la figure 2 se caractérise en ce que les pales de brassage mécanique 5 s'étendent, depuis la paroi externe de la deuxième extrémité 4b de la partie centrale 4, en éloignement de la cavité interne 6 de la partie centrale 4. Il s'agit donc ici de pales de brassage 5 externes.

Au contraire, l'exemple de réalisation de la figure 3 se caractérise en ce que les pales de brassage mécanique 5 s'étendent, depuis la paroi interne de la deuxième extrémité 4b de la partie centrale 4, à l'intérieur de la cavité interne 6 de la partie centrale 4. Il s'agit donc ici de pales de brassage 5 internes.

Toutefois, de façon commune aux deux réalisations et conformément à l'invention, les pales de brassage 5 sont destinées à être totalement immergées dans le bain de silicium 2, comme visible sur la figure 1.

Par ailleurs, sans que cela soit limitatif, chaque dispositif 1 comporte trois pales de brassage mécanique 5 et la partie centrale 4 comporte trois orifices 7 séparés par trois portions de paroi longitudinales 4d, mis en place de manière symétrique par répétition circulaire et à distance constante. Le nombre des pales 5 peut varier en fonction de la vitesse de brassage et de la nature du liquide à brasser.

Dans le cas présent, la vitesse de rotation des pales 5 dépend de la forme des pales 5 et du but à atteindre, et peut être par exemple comprise entre 20 et 25 tours/min.

La hauteur d'introduction dans le bain de silicium 2 correspond avantageusement à une hauteur suffisante pour assurer la remontée du liquide aspiré par la deuxième extrémité 4b centrale creuse vers la surface libre S d'analyse. Ainsi, la hauteur totale Ht des orifices 7, visible sur les figures 1 et 2, peut être de l'ordre de 40 mm, et la hauteur immergée H des orifices 7, visible sur la figure 1, est par exemple de l'ordre de 10 mm.

Par ailleurs, comme visible sur la figure 1, l'enceinte 11 est de forme cylindrique et présente un diamètre interne De, et les pales de brassage mécanique 5 présentent une plus grande dimension transversale assimilable à un diamètre Dp, selon l'axe transversal Y perpendiculaire à l'axe longitudinal X de la partie centrale 4, de l'ordre de quelques centimètres jusqu'à une dizaine de mètres. Ces paramètres De et Dp vérifient la relation suivante: $0,2 < Dp/De < 0,7$.

Selon les deux modes de réalisation des figures 2 et 3, chaque pale de brassage mécanique 5 comporte une première aile de brassage 8a orientée selon un angle d'orientation $\alpha$ qui est de l'ordre de 20° par rapport à l'axe transversal Y perpendiculaire à l'axe longitudinal X de la partie centrale 4, comme visible sur la figure 2.

Chaque pale de brassage 5 comporte également une deuxième aile de brassage 8b, reliée à la première aile de brassage 8a, et présentant une inclinaison plus faible que celle de la première aile de brassage 8a par rapport à l'axe transversal Y. La première aile de brassage 8a comporte le bord d'attaque de la pale de brassage 5 correspondante.

Globalement, la forme de chaque pale de brassage 5 est ainsi légèrement inclinée par rapport à l'horizontal de telle manière que la partie supérieure de la pale 5 et la partie inférieure de la pale 5 soient sensiblement au même niveau que la deuxième extrémité 4b. Autrement dit encore, comme visible sur la figure 2 par exemple, la hauteur Hp de chaque pale de brassage mécanique 5 est sensiblement égale à la hauteur h de la deuxième extrémité 4b de la partie centrale 4. L'invention propose donc ici une faible inclinaison des pales 5 à l'inverse des solutions de l'art antérieur pour lesquelles l'angle d'inclinaison est beaucoup plus important et impose d'avoir une surface de contact avec le liquide qui soit plus grande et en conséquence requiert plus d'énergie pour se déplacer.

Ici, l'inclinaison de la première aile de brassage 8a formant le bord d'attaque impose un déplacement vertical du liquide qui rentre en contact avec ce bord d'attaque.

Selon le premier mode de réalisation de la figure 2, les pales 5 externes permettent de générer un débit axial, soit un mouvement vertical du liquide, au niveau des pales 5 à l'extérieur de la partie centrale 4. Le sens de ce mouvement, vers le bas ou vers le haut, est relié au sens de rotation. La deuxième extrémité 4b creuse permet une aspiration du liquide vers le haut, soit vers la surface d'analyse S, et son évacuation par le biais des orifices 7.

Selon le deuxième mode de réalisation de la figure 3, les pales 5 internes permettent, pour une rotation dans le sens horaire, l'aspiration du liquide à travers la deuxième extrémité 4b creuse vers le haut et plus précisément vers la surface libre d'analyse S et ensuite son évacuation par le biais des orifices 7. La vitesse d'écoulement reste constante pendant le brassage.

Contrairement aux pales de l'art antérieur, le comportement du liquide généré par les deux formes de pales des réalisations des figures 2 et 3 est avantageux et original. En plus du brassage du liquide, la deuxième extrémité 4b creuse permet une aspiration verticale du liquide qui va s'évacuer par le biais des orifices 7 et ainsi renouveler la surface d'analyse S.

Le nombre de pales 5 peut varier en fonction de la puissance et de la vitesse de brassage souhaitées, des propriétés du liquide et de la géométrie de l'enceinte 11.

Le sens de rotation est imposé par la forme des pales 5, et plus précisément des ailes 8a, 8b.

Des essais ont été réalisés dans une enceinte 11 sous forme d'un creuset transparent en utilisant des dimensions similaires à celles du creuset en graphite dans le lequel le silicium, typiquement 3 kg, est fondu dans le four de fusion. Le diamètre extérieur de ce creuset 11 est d'environ 125 mm et le diamètre intérieur est d'environ 115 mm avec une hauteur extérieure d'environ 240 mm et une hauteur intérieur d'environ 228 mm.

Les essais ont été réalisés dans l'eau étant donné que les propriétés physiques de l'eau à 20°C se rapprochent de celles du silicium liquide à 1450°C.

Le suivi de l'écoulement de l'eau a été obtenu en utilisant des traceurs en suspension dans le fluide. Ces traceurs suivent localement le mouvement du liquide.

Dans le cas de pales de brassage 5 internes, comme sur l'exemple de réalisation de la figure 3, la rotation des pales en sens horaire engendre un contact entre les particules de l'eau avec le bord d'attaque des pales inclinées. Ce bord correspond à la partie inférieure de la pale, derrière un glissement des particules du liquide à travers la pale vers le haut entraînant un vide de l'autre côté de la pale qui est rempli par le liquide inférieur. Autrement dit, une aspiration du liquide se trouvant en bas de la pale 5 est induite vers la surface libre S. La présence des orifices 7 permet une évacuation horizontale des particules qui atteignent la surface libre S jusqu'à atteindre les bords puis descendent le long de la paroi de l'enceinte 11 pour rejoindre le centre de celle-ci par le biais d'un mouvement axial. La surface libre S ciblée par le laser reste donc stable, renouvelable et représentative avec une absence de vortex, de zone de turbulence ou de cavité.

Dans le cas de pales de brassage 5 externes, comme sur l'exemple de réalisation de la figure 2, le brassage induit par les pales en rotation en sens antihoraire est caractérisé par un mouvement d'aspiration important en dessous des pales 5 qui induit une remontée d'une grande quantité de particules vers le haut par le biais du creux de la deuxième extrémité 4b. Au niveau des extrémités des pales 5, les particules qui sont en contact avec le bord d'attaque, ont tendance à descendre en glissant sur la pale, ce qui génère un mouvement sous forme de boucle axiale au niveau des pales 5 et impose au fluide de remonter par le biais de la deuxième extrémité 4b de la partie centrale 4 vers la surface libre S et ainsi le renouvellement de la surface d'analyse S. L'écoulement au travers de la deuxième extrémité 4b est avec un nombre de Reynolds de $Re = 1277$, ce qui correspond à écoulement laminaire.

A la fusion complète, les pales 5, fabriquées par exemple en graphite, collées à la partie centrale 4 réalisée en alumine, avec par exemple un diamètre interne de 14 mm et un diamètre externe de 18 mm, sont progressivement introduites dans le bain de silicium 2, à savoir une introduction totale des pales 5 et partielle des orifices 7 selon un mouvement de translation, en utilisant un soufflet à coupelles soudées. Puis, grâce à un passage rotatif creux relié à un moteur excentré, la partie centrale 4 est mise en rotation pour assurer le brassage du bain. Pour éviter toute déviation de l'axe centrale X pendant la rotation qui pourrait endommager l'arbre, un système de centrage est utilisé. La vitesse de rotation du dispositif de brassage 1 est fixée à 25 tours/mn. Ces conditions permettent un écoulement laminaire et une circulation du liquide à travers des orifices 7 prévus dans le tube 4. Ce système de rotation est relié à la tête 3 LIBS sans la rotation de la bride de liaison. La chaîne de mesure LIBS est par exemple composée d'un laser Nd-YAG nano pulsé (durée de pulse de 5 ns) fonctionnant à une longueur d'onde de 1064 nm. Le laser permet de délivrer des pulses d'une énergie maximale de 200 mJ. Le signal est récupéré par le biais d'un détecteur puis focalisé à l'entrée d'un monochromateur à l'aide d'un faisceau de fibres optiques.

Bien entendu, l'invention n'est pas limitée aux exemples de réalisation qui viennent d'être décrits. Diverses modifications peuvent y être apportées par l'homme du métier.

La géométrie des pales de brassage 5, par exemple le diamètre, l'inclinaison des ailes, le nombre des ailes, les dimensions des orifices 7, le nombre d'orifices 7, la forme des orifices 7 et les paramètres de brassage, tels que vitesse de rotation, hauteur d'introduction des pales 5, sens de rotation, pourront être adaptés en fonction de la nature du matériau à analyser et la géométrie de l'enceinte 11.

L'invention peut en outre être utilisée pour différents liquides, tels que de l'eau, des métaux liquides, entre autres, et à différentes températures, notamment température ambiante ou hautes températures.

Les pales 5 peuvent être fabriquées en graphite ou à base de n'importe quel matériau qui peut résister à l'environnement auquel elles sont soumises. Par exemple, si les pales 5 sont utilisées à hautes températures, ce matériau doit avoir une bonne réfractarité en fonction de la température du bain, une bonne résistance aux chocs thermiques étant donné que les pales sont introduites puis retirées du bain à plusieurs reprises et une bonne résistance à l'abrasion et à l'érosion en fonction de la nature du liquide. A température ambiante, les pales 5 peuvent être fabriquées par exemple en polyamide.

Pour des applications à hautes températures, la partie centrale 4 peut être fabriquée en un matériau qui doit, en plus de sa résistance aux contraintes thermomécaniques de l'environnement, être isolant thermiquement afin de ne pas détériorer les moyens d'analyse LIBS. Par exemple, la partie centrale 4 peut être fabriquée en alumine.

La liaison entre la partie centrale 4 et les pales 5 peut être réalisée par collage, filetage et/ou pas de vis.

Par ailleurs, les moyens d'analyse LIBS peuvent comporter un laser apte à générer une impulsion laser vers la surface à analyser du bain de silicium 2, un jeu de miroirs permettant la focalisation de l'impulsion laser vers la surface à analyser, un dispositif de collecte des émissions du micro-plasma formé par l'impulsion laser relié à une fibre optique, et un spectromètre d'émission permettant l'analyse des émissions collectées.

La durée de l'impulsion du laser peut être de l'ordre de la femtoseconde à la nanoseconde. De plus, le laser peut fonctionner à différentes longueurs d'onde, par exemple comprises entre 266 nm et 1064 nm, et préférentiellement dans le domaine de l'infrarouge. Son énergie peut être supérieure à 100 mJ.

Le jeu de miroirs peut permettre la focalisation de l'impulsion laser vers la surface à analyser à une distance d'environ 2 m.

La focalisation de l'impulsion laser vers la surface à analyser peut permettre la création du micro-plasma dont les émissions sont collectées. Le spectromètre d'émission, qui permet alors d'analyser les émissions collectées, peut par exemple être un monochromateur du type « Czerny-Turner », pourvu de réseaux de diffraction adaptés.

En outre, le dispositif d'analyse 1 selon l'invention peut comporter un système de soufflage de gaz inerte, notamment de l'hélium ou de l'argon, au travers de la partie centrale 4.

De façon avantageuse, le soufflage de gaz inerte à l'intérieur de la partie centrale 4 peut permettre d'éviter d'éventuelles contaminations de la surface à analyser, par exemple pour éviter une oxydation en cas de micro-fuites. En outre, le soufflage de gaz inerte peut également présenter l'avantage d'augmenter la sensibilité de mesures et diminuer les limites de détection de l'analyse par la technique de spectroscopie.

**Revendications**

1.	Dispositif (1) de caractérisation d'au moins un matériau liquide (2), comportant :

	- des moyens de caractérisation (3),
	un bain liquide dudit matériau liquide (2),
	- des moyens de brassage mécanique (4, 5) rotatifs dudit bain liquide dudit au moins un matériau liquide (2), les moyens de brassage mécanique (4, 5) comportant :

		- une partie centrale (4), s'étendant selon un axe longitudinal (X), destinée à être positionnée au-dessus du bain liquide dudit au moins un matériau liquide (2), comprenant une cavité interne (6) formant une chambre d'analyse délimitée par une paroi (4c) de la partie centrale (4), la partie centrale (4) comportant une première extrémité (4a) reliée aux moyens de caractérisation (3), et
		- une pluralité de pales de brassage mécanique (5), reliées à une deuxième extrémité (4b) de la partie centrale (4), opposée à la première extrémité (4a) de la partie centrale (4), la deuxième extrémité (4b) de la partie centrale (4) s'étendant sur une hauteur (h) selon l'axe longitudinal (X) de la partie centrale (4) et étant creuse de sorte à assurer une communication fluidique entre la cavité interne (6) et le bain liquide dudit au moins un matériau liquide (2),

	les moyens de caractérisation (3) étant configurés pour permettre l'analyse de la surface (S) dudit au moins un matériau liquide (2), situé dans la portion au droit de la cavité interne (6) de la partie centrale (4),
	**caractérisé en ce que** les pales de brassage mécanique (5) sont totalement immergées dans le bain liquide dudit au moins un matériau liquide (2), **en ce que** la partie centrale (4) comporte un ou plusieurs orifices (7) formés de façon traversante dans sa paroi (4c) délimitant la cavité interne (6) et situés au-dessus de la deuxième extrémité (4b) de la partie centrale (4) portant les pales de brassage mécanique (5) lorsque le dispositif (1) est en place dans le bain liquide dudit au moins un matériau liquide (2), les orifices (7) étant partiellement immergés selon une hauteur (H) dans le bain liquide dudit au moins un matériau liquide (2),
	et **en ce que** chaque pale de brassage mécanique (5) comporte au moins une aile de brassage (8a, 8b) orientée selon un angle d'orienta-

tion ($\alpha$) non nul par rapport à un axe transversal (Y) perpendiculaire à l'axe longitudinal (X) de la partie centrale (4).

2. Dispositif selon la revendication 1, **caractérisé en ce qu'**il est configuré pour l'analyse d'au moins un métal (2) en fusion oxydable par technique LIBS, les moyens de caractérisation étant des moyens d'analyse par technique de spectroscopie (3) comprenant des moyens d'analyse par technique LIBS.

3. Dispositif selon la revendication 1 ou 2, **caractérisé en ce que** les pales de brassage mécanique (5) s'étendent, depuis la paroi externe de la deuxième extrémité (4b) de la partie centrale (4), en éloignement de la cavité interne (6) de la partie centrale (4).

4. Dispositif selon la revendication 1 ou 2, **caractérisé en ce que** les pales de brassage mécanique (5) s'étendent, depuis la paroi interne de la deuxième extrémité (4b) de la partie centrale (4), à l'intérieur de la cavité interne (6) de la partie centrale (4).

5. Dispositif selon l'une quelconque des revendications précédentes, **caractérisé en ce que** la hauteur (Hp) de chaque pale de brassage mécanique (5), selon l'axe longitudinal (X) de la partie centrale (4), est sensiblement égale à la hauteur (h), selon l'axe longitudinal (X) de la partie centrale (4), de la deuxième extrémité (4b) de la partie centrale (4).

6. Dispositif selon l'une quelconque des revendications précédentes, **caractérisé en ce que** l'angle d'orientation ($\alpha$) est inférieur ou égal à 20°.

7. Dispositif selon l'une quelconque des revendications précédentes, **caractérisé en ce que** les moyens de brassage mécanique (4, 5) comportent trois pales de brassage mécanique (5) et **en ce que** la partie centrale (4) comporte trois orifices (7) séparés les uns des autres par des portions de paroi longitudinales (4d).

8. Dispositif selon l'une quelconque des revendications précédentes, **caractérisé en ce que** le ou les orifices (7) sont destinés à être partiellement immergés selon une hauteur (H) dans le bain liquide dudit au moins un matériau liquide (2), au moins égale à un quart de la hauteur totale (Ht) du ou des orifices (7).

9. Dispositif selon l'une quelconque des revendications précédentes, **caractérisé en ce que** la partie centrale (4) se présente sous la forme d'un tube creux, notamment sous la forme d'un tube cylindrique creux.

10. Ensemble (10), **caractérisé en ce qu'**il comporte :

- une enceinte (11) comportant un bain d'au moins un matériau liquide (2),
- un dispositif (1) de caractérisation selon l'une quelconque des revendications précédentes, pour la caractérisation dudit au moins un matériau liquide (2) de l'enceinte (11).

11. Ensemble selon la revendication 10, **caractérisé en ce que** ledit au moins un matériau liquide (2) est un métal en fusion, notamment un métal fortement oxydable, étant notamment choisi parmi le silicium ou le zirconium.

12. Ensemble selon la revendication 10 ou 11, **caractérisé en ce que** l'enceinte (11) et la partie centrale (4) du dispositif (1) de caractérisation sont réalisées dans un même matériau, notamment en graphite.

13. Ensemble selon l'une quelconque des revendications 10 à 12, **caractérisé en ce que** l'enceinte (11) est de forme cylindrique présentant un diamètre interne (De), **en ce que** les pales de brassage mécanique (5) présentent une plus grande dimension transversale (Dp), notamment un diamètre, selon un axe transversal (Y) perpendiculaire à l'axe longitudinal (X) de la partie centrale (4), et **en ce que** la relation suivante est vérifiée :

$$0{,}2 < Dp/De < 0{,}7,$$

où :

Dp est la plus grande dimension transversale des pales de brassage mécanique (5), et
De est le diamètre interne de l'enceinte (11).

14. Procédé de caractérisation d'au moins un matériau liquide (2), **caractérisé en ce qu'**il est mis en oeuvre au moyen d'un dispositif (1) de caractérisation selon l'une quelconque des revendications 1 à 9, et **en ce qu'**il comporte les étapes simultanées consistant à :

- effectuer un brassage mécanique d'un bain liquide dudit au moins un matériau liquide (2) par le biais des moyens de brassage mécanique (4, 5) rotatifs du dispositif (1),
- analyser la surface (S) dudit au moins un matériau liquide (2) situé au droit de la cavité interne (6) de la partie centrale (4) par le biais des moyens de caractérisation (3).

15. Procédé selon la revendication 14, **caractérisé en ce que** ledit au moins un matériau liquide (2) est un métal en fusion oxydable, notamment un métal fortement oxydable, étant notamment choisi parmi le silicium ou le zirconium, **en ce que** les moyens de caractérisation sont des moyens d'analyse par technique de spectroscopie (3) comprenant des moyens d'analyse par technique LIBS, et **en ce que** le procédé comporte au moins une étape d'analyse en ligne par technique LIBS d'une ou plusieurs impuretés contenues dans ledit au moins un métal (2) en fusion oxydable, notamment du silicium, lors d'un processus de purification dudit au moins un métal (2) en fusion oxydable.

**Patentansprüche**

1. Vorrichtung (1) zur Charakterisierung von mindestens einem flüssigen Material (2), beinhaltend:

   - Charakterisierungsmittel (3),
   - ein Flüssigkeitsbad des flüssigen Materials (2),
   - rotierende mechanische Rührmittel (4, 5) des Flüssigkeitsbads des mindestens einen flüssigen Materials (2), wobei die mechanischen Rührmittel (4, 5) beinhalten:

      - einen Mittelteil (4), der sich entlang einer Längsachse (X) erstreckt, und bestimmt ist, oberhalb des Flüssigkeitsbads des mindestens einen flüssigen Materials (2) positioniert zu werden, einen inneren Hohlraum (6) umfassend, der eine Analysekammer bildet, die durch eine Wand (4c) des Mittelteils (4) begrenzt ist, wobei der Mittelteil (4) ein erstes Ende (4a) beinhaltet, das mit den Charakterisierungsmitteln (3) verbunden ist, und
      - eine Vielzahl von mechanischen Rührblättern (5), die mit einem zweiten Ende (4b) des Mittelteils (4) verbunden sind, das dem ersten Ende (4a) des Mittelteils (4) gegenüberliegt, wobei sich das zweite Ende (4b) des Mittelteils (4) in einer Höhe (h) entlang der Längsachse (X) des Mittelteils (4) erstreckt, und hohl ist, um eine Fluidverbindung zwischen dem inneren Hohlraum (6) und dem Flüssigkeitsbad des mindestens einen flüssigen Materials (2) zu gewährleisten,
      wobei die Charakterisierungsmittel (3) konfiguriert sind, um die Analyse der Oberfläche (S) des mindestens einen flüssigen Materials (2) zu ermöglichen, das sich im Abschnitt rechtwinklig zum inneren Hohlraum (6) des Mittelteils (4) befindet, **dadurch gekennzeichnet, dass** die mechanischen Rührblätter (5) vollständig in das Flüssigkeitsbad des mindestens einen flüssigen Materials (2) eingetaucht sind, dass der Mittelteil (4) eine oder mehrere Öffnungen (7) beinhaltet, die durch seine Wand (4c) durchgehend ausgebildet sind, die den inneren Hohlraum (6) begrenzt, und sich oberhalb des zweiten Endes (4b) des Mittelteils (4) befinden, der die mechanischen Rührblätter (5) trägt, wenn die Vorrichtung (1) im Flüssigkeitsbad des mindestens einen flüssigen Materials (2) angeordnet ist, wobei die Öffnungen (7) gemäß einer Höhe (H) teilweise eingetaucht sind im Flüssigkeitsbad des mindestens einen flüssigen Materials (2),
   und dass jedes mechanische Rührblatt (5) mindestens einen Rührflügel (8a, 8b) beinhaltet, der in einem Orientierungswinkel ($\alpha$) ungleich null zu einer Querachse (Y) senkrecht zur Längsachse (X) des Mittelteils (4) ausgerichtet ist.

2. Vorrichtung nach Anspruch 1, **dadurch gekennzeichnet, dass** sie für die Analyse von mindestens einem geschmolzenen oxidierbaren Metall (2) mittels LIBS-Technik konfiguriert ist, wobei die Charakterisierungsmittel Analysemittel mittels Spektroskopietechnik (3) sind, Analysemittel mittels LIBS-Technik umfassend.

3. Vorrichtung nach Anspruch 1 oder 2, **dadurch gekennzeichnet, dass** sich die mechanischen Rührblätter (5) von der Außenwand des zweiten Endes (4b) des Mittelteils (4) aus weg vom inneren Hohlraum (6) des Mittelteils (4) erstrecken.

4. Vorrichtung nach Anspruch 1 oder 2, **dadurch gekennzeichnet, dass** sich die mechanischen Rührblätter (5) von der Innenwand des zweiten Endes (4b) des Mittelteils (4) aus in den inneren Hohlraum (6) des Mittelteils (4) erstrecken.

5. Vorrichtung nach einem der vorhergehenden Ansprüche, **dadurch gekennzeichnet, dass** die Höhe (Hp) jedes mechanischen Rührblatts (5) entlang der Längsachse (X) des Mittelteils (4) im Wesentlichen gleich der Höhe (h) entlang der Längsachse (X) des Mittelteils (4) des zweiten Endes (4b) des Mittelteils (4) ist.

6. Vorrichtung nach einem der vorhergehenden Ansprüche, **dadurch gekennzeichnet, dass** der Orientierungswinkel ($\alpha$) kleiner oder gleich 20° ist.

7. Vorrichtung nach einem der vorhergehenden Ansprüche, **dadurch gekennzeichnet, dass** die mechanischen Rührmittel (4, 5) drei mechanische Rührblätter (5) beinhalten und dass der Mittelteil (4) drei durch Längswandabschnitte (4d) voneinander getrennte Öffnungen (7) beinhaltet.

8. Vorrichtung nach einem der vorhergehenden Ansprüche, **dadurch gekennzeichnet, dass** die Öffnung oder Öffnungen (7) dazu bestimmt sind, teilweise gemäß einer Höhe (H) in das Flüssigkeitsbad des mindestens einen flüssigen Materials (2) eingetaucht zu werden, die mindestens einem Viertel der Gesamthöhe (Ht) der Öffnung oder Öffnungen (7) entspricht.

9. Vorrichtung nach einem der vorhergehenden Ansprüche, **dadurch gekennzeichnet, dass** der Mittelteil (4) in Form eines hohlen Rohrs, insbesondere in Form eines hohlen zylindrischen Rohrs vorliegt.

10. Baugruppe (10), **dadurch gekennzeichnet, dass** sie beinhaltet:

   - einen Behälter (11), der ein Bad mit mindestens einem flüssigen Material (2) beinhaltet,
   - eine Charakterisierungsvorrichtung (1) nach einem der vorhergehenden Ansprüche zur Charakterisierung des mindestens einen flüssigen Materials (2) des Behälters (11).

11. Baugruppe nach Anspruch 10, **dadurch gekennzeichnet, dass** das mindestens eine flüssige Material (2) ein geschmolzenes Metall, insbesondere ein stark oxidierbares Metall ist, insbesondere ausgewählt aus Silizium oder Zirkonium.

12. Baugruppe nach Anspruch 10 oder 11, **dadurch gekennzeichnet, dass** der Behälter (11) und der Mittelteil (4) der Charakterisierungsvorrichtung (1) aus demselben Material bestehen, insbesondere aus Grafit.

13. Baugruppe nach einem der Ansprüche 10 bis 12, **dadurch gekennzeichnet, dass** der Behälter (11) zylindrisch geformt ist und einen Innendurchmesser (De) aufweist, dass die mechanischen Rührblätter (5) eine größere Querabmessung (Dp) aufweisen, insbesondere einen Durchmesser, entlang einer Querachse (Y) senkrecht zur Längsachse (X) des Mittelteils (4), und dass das folgende Verhältnis verifiziert ist:

$$0,2 < Dp/De < 0,7,$$

wobei:

   Dp die größte Querabmessung der mechanischen Rührblätter (5) ist, und
   De der Innendurchmesser des Behälters (11) ist.

14. Verfahren zur Charakterisierung von mindestens einem flüssigen Material (2), **dadurch gekennzeichnet, dass** es mittels einer Charakterisierungsvorrichtung (1) nach einem der Ansprüche 1 bis 9 durchgeführt wird, und dass es die gleichzeitigen Schritte beinhaltet, die bestehen aus:

   - mechanisches Rühren eines Flüssigkeitsbads des mindestens einen flüssigen Materials (2) durch die rotierenden mechanischen Rührmittel (4, 5) der Vorrichtung (1),
   - Analysieren der Oberfläche (S) des mindestens einen flüssigen Materials (2), das sich rechtwinklig zum inneren Hohlraum (6) des Mittelteils (4) befindet, durch die Charakterisierungsmittel (3).

15. Verfahren nach Anspruch 14, **dadurch gekennzeichnet, dass** das mindestens eine flüssige Material (2) ein geschmolzenes oxidierbares Metall ist, insbesondere ein stark oxidierbares Metall, insbesondere ausgewählt aus Silizium oder Zirkonium, dass die Charakterisierungsmittel Analysemittel durch Spektroskopietechnik (3) sind, Analysemittel der LIBS-Technik umfassend, und dass das Verfahren mindestens einen Schritt der Online-Analyse durch LIBS-Technik von einer oder mehreren Verunreinigungen umfasst, die in dem mindestens einen geschmolzenen oxidierbaren Metall (2), insbesondere Silizium, enthalten sind, bei einem Reinigungsprozess des mindestens einen geschmolzenen oxidierbaren Metalls (2).

## Claims

1. Device (1) for characterising at least one liquid material (2), comprising:

   - characterisation means (3),
   A liquid melt of said liquid material (2),
   - rotating means for mechanically stirring (4, 5) said liquid melt of said at least one liquid material (2), the mechanical stirring means (4, 5) comprising:

      - a central part (4), extending along a longitudinal axis (X), intended to be positioned above the liquid melt of said at least one liquid material (2), comprising an inner cavity (6) forming an analysis chamber delimited by a wall (4c) of the central part (4), 10 the central part (4) comprising a first end (4a) connected to the characterisation means (3), and
      - a plurality of mechanical stirring blades (5), connected to a second end (4b) of the central part (4), opposite to the first end (4a) of the central part (4), the second end (4b) of the central part (4) extending over a height (h) along the longitudinal axis (X) of the central part (4) and being hollow so as to ensure fluidic communication between the inner cavity (6) and the liquid melt of said at least one liquid material (2), the characterisation means (3) being configured to enable the analysis of the surface (S) of said at least one liquid material (2), situated in the portion directly in line with the inner cavity (6) of the central part (4), **characterised in that** the mechanical stirring blades (5) are totally immersed in the liquid melt of said at least one liquid material (2), **in that** the central part (4) comprises one or more orifices (7) formed in a traversal manner in its wall (4c) delimiting the inner cavity (6) and situated above the second end (4b) of the central part (4) bearing the mechanical stirring blades (5) when the device (1) is in place in the liquid melt of said at least one liquid material (2), orifices (7) being partially immersed according to a height (H) in the liquid melt of said at least one liquid material (2), and **in that** each mechanical stirring blade (5) comprises at least one stirring wing (8a, 8b) oriented according to a non-zero angle of orientation (a) with respect to a transversal axis (Y) perpendicular to the longitudinal axis (X) of the central part (4).

2. Device according to claim 1, **characterised in that** it is configured for the analysis of at least one oxidisable molten metal (2) by LIBS technique, the characterisation means being means for analysing by spectroscopy technique (3) comprising means for analysing by LIBS technique.

3. Device according to claim 1 or 2, **characterised in that** the mechanical stirring blades (5) extend, from the outer wall of the second end (4b) of the central part (4), while moving away from the inner cavity (6) of the central part (4).

4. Device according to claim 1 or 2, **characterised in that** the mechanical stirring blades (5) extend, from the inner wall of the second end (4b) of the central part (4), inside the inner cavity (6) of the central part (4).

5. Device according to any one of the preceding claims, **characterised in that** the height (Hp) of each mechanical stirring blade (5), along the longitudinal axis (X) of the central part (4), is substantially equal to the height (h), along the longitudinal axis (X) of the central part (4), of the second end (4b) of the central part (4).

6. Device according to any one of the preceding claims, **characterised in that** the angle of orientation (a) is less than or equal to 20 °.

7. Device according to any one of the preceding claims, **characterised in that** the mechanical stirring means (4, 5) comprise three mechanical stirring blades (5) and **in that** the central part (4) comprises three orifices (7) separated from each other by longitudinal wall portions (4d).

8. Device according to any one of the preceding claims, **characterised in that** the orifice or orifices (7) are intended to be partially immersed according to a height (H) in the liquid melt of said at least one liquid material (2), at least equal to a quarter of the total height (Ht) of the orifice or orifices (7).

9. Device according to any one of the preceding claims, **characterised in that** the central part (4) is in the form of a hollow tube, notably in the form of a hollow cylindrical tube.

10. Assembly (10), **characterised in that** it comprises:

    - a vessel (11) comprising a melt of at least one liquid material (2),
    - a characterisation device (1) according to any one of the preceding claims, for the characterisation of said at least one liquid material (2) of the vessel (11).

11. Assembly according to claim 10, **characterised in that** said at least one liquid material (2) is a molten metal, notably a highly oxidisable metal, being notably selected from silicon or zirconium.

12. Assembly according to claim 10 or 11, **characterised in that** the vessel (11) and the central part (4) of the characterisation device (1) are made of a same material, notably graphite.

13. Assembly according to any one of claims 10 to 12, **characterised in that** the vessel (11) is of cylindrical shape having an inner diameter (De), **in that** the mechanical stirring blades (5) have a larger transversal dimension (Dp), notably a diameter, along a transversal axis (Y) perpendicular to the longitudinal axis (X) of the central part (4),

    and **in that** the following relationship is verified:

    $$0.2 < Dp/De < 0.7,$$

    where:

    Dp is the largest transversal dimension of the mechanical stirring blades (5), and
    De is the inner diameter of the vessel (11).

14. Method for characterising at least one liquid material (2), **characterised in that** it is implemented by means of a characterisation device (1) according to any one of claims 1 to 9, and **in that** it comprises the simultaneous steps consisting in:

- carrying out a mechanical stirring of a liquid melt of said at least one liquid material (2) by means of rotating mechanical stirring means (4, 5) of the device (1),
- analysing the surface (S) of said at least one liquid material (2) situated directly in line with the inner cavity (6) of the central part (4) by means of characterisation means (3).

15. Method according to claim 14, **characterised in that** said at least one liquid material (2) is an oxidisable molten metal, notably a highly oxidisable metal, being notably selected from silicon or zirconium, **in that** the characterisation means are means for analysing by spectroscopy technique (3) comprising means for analysing by LIBS technique, and **in that** the method comprises at least one step of on-line analysis by LIBS

technique of one or more impurities contained in said at least one oxidisable molten metal (2), notably silicon, during a process of purification of said at least one oxidisable molten metal (2).

FIG. 1

FIG. 2

FIG. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02063284 A2
- US 20030234928 A1
- FR 3021407 A1
- US 4717540 A
- US 8281964 B2
- US 4802656 A

**Littérature non-brevet citée dans la description**

- **SARAH DARWICHE et al.** Impurity détection in solid and molten silicon by laser induced breakdown spectroscopy. *Spectrochimica Acta,* 2012, vol. 74 (75), 115-118
- **AWADHESH K. RAI et al.** High température fiber optic laser-induced breakdown spectroscopy sensor for analysis of molten alloy constituents. *Review of Scientific Instruments,* Octobre 2002, vol. 73 (10), 3589-3599
- **N. RAMASEDER et al.** VAI-CON® Chem-A New Continuous Chemical Analysis System of Liquid Steel in Metallurgical Vessels. *La metallurgia italiana,* Février 2004, 60-63